# EUROPEAN PATENT APPLICATION

(11) **EP 1 798 279 A1**
(43) Date of publication of application: **20.06.2007**
(21) Application number: 05027589.0
(22) Date of filing: 16.12.2005
(51) Int. Cl.: C11D 3/382, A61K 8/97

(54) **Pre-mixes for personal and home care compositions**

(71) Applicant: Cognis IP Management GmbH, 40589 Düsseldorf (DE)
(72) Inventor: Corbella, Alberto, 22100 Como (IT); Domingo, Marta, 08024 Barcelona (ES); Triggiani, Fabio, 22073 Como (IT); Gargano, Salvatore, 08034 Barcelona (ES); Martinez, Silvia, c/Aribau, 08173 St.Cugat des Vallés (Barcelona) (ES); Pettinari, Gabriele, 21013 Gallarate (IT)
(74) Representative: Fabry, Bernd

(57) **Abstract**

Suggested are new pre-mixes for personal and home care compositions, consisting of
(a) 5 to 70 % b.w. anionic, non-ionic and/or amphoteric surfactants,
(b) 0.01 to 10 % b.w. botanical extracts, and
(c) 0 to 1 % b.w. preservatives

under the condition that the amounts add with water to 100 % b.w.

## Description

### Object of the invention

The present invention relates to aqueous pre-mixes for making personal or home care compositions, consisting of surfactants, plant extracts, and optionally, preservatives, and the use of said pre-mixtures for making various types of compositions.

### State of the art

Personal and home care compositions are well-known and widely used. Also known for a long time are botanicals and the benefits they can provide within various detergent compositions. For example, DE 10251856 A1 and DE 10253976 A1 (both of Cognis) disclose detergent and textile treatment compositions comprising anionic, non-ionic or cationic surfactants in combination with extracts of green tea or olive leaves or their active principles, respectively. Usually, plant extracts are sold either as spray-dried powders or diluted in organic solvents such as propylenglycol, butylenglycol or glycerin. The latter is preferred due to the fact that liquid compositions are much easier to mix with other ingredients of a complex formulation. This, however, shows several serious disadvantages: firstly, the composition of the liquid extracts in terms of active ingredients is not controlled; secondly, the active ingredients are present in such low concentrations that it is difficult to prove the presence of the extracts in the final formulations; thirdly, the extracts are often coloured and exhibit limited storage stability towards colour and odour depending on the concentration of use, and finally, organic solvents are usually less preferred and sometimes undesired for making certain products which come into contact with the human skin.

Therefore, the complex problem underlying the present invention has been to develop a composition for offering botanical extracts, which avoids the disadvantages of the state of the art cited above. Particularly, said compositions should be standardised, have a batch to batch consistency, be free of organic solvents, exhibit higher storage stability with respect to colour and odour of the plant extracts, providing additional benefits to the final personal or home care compositions.

### Description of the invention

The present invention claims new pre-mixes for personal and home care compositions, essentially consisting of
(a) 5 to 70, preferably 10 to 50, and more preferably, 20 to 40 % b.w. anionic, non-ionic and/or amphoteric surfactants,
(b) 0.01 to 10, preferably 0.5 to 8, and more preferably, 1 to 5 % b.w. botanical extracts, and
(c) 0 to 1, preferably 0.1 to 0.5 % b.w. preservatives
under the condition that the amounts add with water to 100 % b.w.

The pre-mixes according to the present invention meet the needs of the market in that they are liquid without comprising any organic solvent and allow a better stability in the final formulation for the customer in terms of colour and odour while reducing the amount of thickeners necessary to adjust a viscosity sufficient for the desired application. In addition, the extracts are standardised having a batch to batch consistency in terms of active ingredients.

### Anionic surfactants

Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homologue distribution although they preferably have a narrow-range homologue distribution.

### • Alk(en)yl ether sulfates

The preferred anionic surfactants, however, are represented by the group of alk(en)yl polyalkylene glycol ether sulfates ("ether sulfates") which are industrially produced by the sulfation of fatty alcohol or oxoalcohol polyglycol ethers with SO₃ or chlorosulfonic acid (CSA) and subsequent neutralization. Alk(en)yl ether sulfates suitable for the purposes of the invention correspond to formula **(I):**

**R¹(OCH₂CHR²)ₙOSO₃X** (I)

where R¹ is a linear or branched alkyl and/or alkenyl group containing 6 to 22, more particularly 12 to 18 carbon atoms, R² is hydrogen or methyl, n is a number from 1 to 5 and X is alkali, alkaline earth, ammonium, alkylammonium, alkanolammonium or glucammonium. Typical examples are the sulfates of addition products of on average 1 to 5 and, more particularly, 2 to 3 moles of ethylene oxide onto caproic alcohol, caprylic alcohol, 2-ethyl hexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and technical mixtures thereof in the form of their alkyl, preferably monoisopropanolammonium salts. The ether sulfates may have both a conventional homologue distribution and a narrow homologue distribution. It is particularly preferred to use ether sulfates based on addition products of, on average, 2 to 3 moles of ethylene oxide with technical C₁₂-C₁₈, preferably C₁₂-C₁₄ fatty alcohol fractions preferably in the form of their sodium, magnesium, ammonium or mono isopropanolammonium salts.

### Non-ionic surfactants

Typical examples for non-ionic surfactants are the following:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- C₄-C₂₂ alk(en)yl oligoglycosides;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol,-dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations.

### • Alk(en)yl polyalkylenglycol ethers

In a preferred embodiment of the present invention non-ionic surfactants of the alk(en)yl polyalkylene glycol ethers type are used, which are industrially produced by the alkoxylation of saturated and/or insaturated fatty alcohols or oxoalcohols with ethylene oxide, propylene oxide or their mixtures. Alkyl polyalkylene glycol ethers suitable for the purposes of the invention correspond to formula **(II):**

**R³(OCH₂CHR⁴)ₘOH** (II)

in which R³ is a linear or branched alkyl and/or alkenyl group containing 6 to 22 carbon atoms, R⁴ is hydrogen or methyl, and m is a number from 1 to 20. Typical examples are the addition products of on average 1 to 20 and, more particularly, 5 to 10 moles of ethylene oxide and/or propylene oxide onto caproic alcohol, caprylic alcohol, 2-ethyl hexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and technical mixtures thereof. The ethers may have both a conventional homologueu distribution and a narrow homologue distribution. It is particularly preferred to use alkyl polyalkylene glycol ethers based on addition products of, on average, 5 to 10, and more particularly of about 7 moles of ethylene oxide with technical C₁₂-C₁₈, preferably C₁₂-C₁₄ fatty alcohol fractions.

### • Alk(en)yl oligoglycosides

In another preferred embodiment of the present invention, non-ionic surfactants of the alkyl or alkenyl oligoglycoside type are used, which may be derived from aldoses or ketoses containing 5 or 6 carbon atoms, preferably glucose. Accordingly, the preferred alkyl and/or alkenyl oligoglycosides are alkyl or alkenyl oligo**glucosides**. These materials are also known generically as "alkyl polyglycosides" (APG). The alk(en)yl oligoglycosides according to the invention preferably correspond to formula **(III):**

**R⁵O[G]ₚ** (III)

where R⁵ is an alkyl or alkenyl radical having 6 to 22 carbon atoms, G is a sugar unit having 5 or 6 carbon atoms and p is a number from 1 to 10. The index p in general formula **(III)** indicates the degree of oligomerisation (DP degree), i.e. the distribution of mono- and oligoglycosides, and is a number from 1 to 10. Whereas p in a given compound must always be an integer and, above all, may assume a value of 1 to 6, the value p for a certain alkyl oligoglycoside is an analytically determined calculated quantity which is mostly a fractional number. Alk(en)yl oligoglycosides having an average degree of oligomerisation p of 1.1 to 3.0 are preferably used. Alk(en)yl oligoglycosides having a degree of oligomerisation below 1.7 and, more particularly, between 1.2 and 1.4 are preferred from the applicational point of view. The alkyl or alkenyl radical R⁵ may be derived from primary alcohols containing 4 to 22 and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides based on hydrogenated C₈-C₁₆ coconut oil alcohol having a DP of 1 to 3 are preferred.

### Amphoteric surfactants

Amphoteric (sometimes also called "zwitterionic") surfactants can be divided into several groups, like alkyl betaines, alkylamido betaines, imidazolins and amphoglycinates.

### • Alkyl betaines

The betaines are known surfactants which are mainly produced by carboxyalkylation, preferably carboxymethylation, of amine compounds. The starting materials are preferably condensed with halocarboxylic acids or salts thereof, more particularly sodium chloroacetate, one mole of salt being formed per mole of betaine. The addition of unsaturated carboxylic acids, such as acrylic acid, for example, is also possible. Examples of suitable betaines are the carboxyalkylation products of secondary and, in particular, tertiary amines which correspond to formula **(IV):** 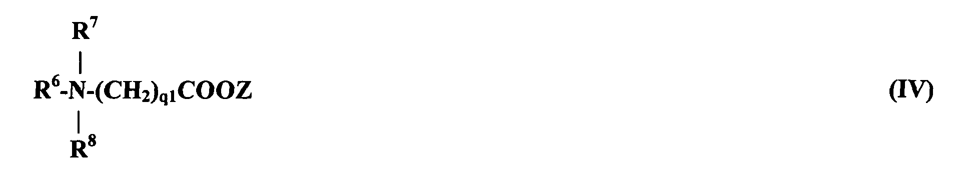 where R⁶ is a an alkyl radical having 6 to 22 carbon atoms, R⁷ is hydrogen or an alkyl group containing 1 to 4 carbon atoms, R⁸ is an alkyl group containing 1 to 4 carbon atoms, q1 is a number of 1 to 6 and X is an alkali and/or alkaline earth metal or ammonium. Typical examples are the carboxymethylation products of hexylmethylamine, hexyldimethylamine, octyldimethylamine, decyldimethylamine, C_{12/14}-cocoalkyldimethylamine, myristyldimethylamine, cetyldimethylamine, stearyldimethylamine, stearylethylmethylamine, oleyldimethylamine, C_{16/18}-tallowalkyldimethylamine and their technical mixtures, and particularly dodecyl methylamine, dodecyl dimethylamine, dodecyl ethylmethylamine and technical mixtures thereof. The commercially available products include Dehyton^{®} AB (Cognis Deutschland GmbH & Co., KG)

### • Alkylamido betaines

Other suitable betaines are the carboxyalkylation products of amidoamines which correspond to formula **(V):** 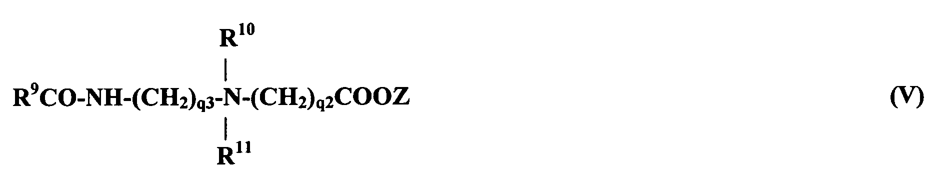 in which R⁹CO is an aliphatic acyl radical having 6 to 22 carbon atoms and 0 or 1 to 3 double bonds, R¹⁰ is hydrogen or an alkyl radical having 1 to 4 carbon atoms, R¹¹ is an alkyl radical having 1 to 4 carbon atoms, q2 is a number from 1 to 6, q3 is a number from 1 to 3 and Z is an alkali and/or alkaline earth metal or ammonium. Typical examples are reaction products of fatty acids having 6 to 22 carbon atoms, for example caproic acid, caprylic acid, caprinic acid, lauric acid, myristic acid, palmitic acid, palmoleic acid, stearic acid, isostearic acid, oleic acid, elaidic acid, petroselinic acid, linolic acid linoleic acid, elaeostearic acid, arachidonic acid, gadoleic acid, behenic acid, erucic acid and their technical mixtures with N,N-dimethylaminoethylamine, N,N-dimethylaminopropylamine, N,N-diethylaminoethylamine und N,N-diethylaminopropylamine, which are condensed with sodium chloroacetate. The commercially available products include Dehyton^{®} K and Dehyton^{®} PK (Cognis Deutschland GmbH & Co. KG) as well as Tego^{®}Betaine (Gold-schmidt).

### • Imidazolines

Other suitable starting materials for the betaines to be used for the purposes of the invention are imidazolines. These substances are also known and may be obtained, for example, by cyclizing condensation of 1 or 2 moles of C₆⁻C₂₂ fatty acids with polyfunctional amines, such as for example aminoethyl ethanolamine (AEEA) or diethylenetriamine. The corresponding carboxyalkylation products are mixtures of different open-chain betaines. Typical examples are condensation products of the above- mentioned fatty acids with AEEA, preferably imidazolines based on lauric acid, which are subsequently betainised with sodium chloroacetate. The commercially available products include Dehyton^{®} G (Cognis Deutschland GmbH & Co. KG). Another suitable amphoteric is Cocamidopropyl Hydroxy Sultaine.

### Botanical extracts

Typically, the plant extracts according to the present invention are chosen from the plants which may have interest for the Personal and Home Care industry such as *Glycine max, Ginkgo biloba, Camellia sinensis, Olea europaea, Glyzyrrhiza glabra, Vaccinium myrtillus, Trifolium pratense, Litchi sinensis, Vitis vinifera, Brassica oleracea, Punica granatum, Petroselinium crispum, Centella asiatica, Passiflora incarnata, Medicago sativa, Valeriana officinalis, Aesculus hippocastanum, Citrus aurantium amara, Salix alba, Hapagophytum procumbens, Calendula officinalis, Chamomila recutita, Panax ginsing, Rosa canina, White musc* and their mixtures. In the following a short summary of the composition and the main constituents of the major cited extracts is given.

### • Ginkgo biloba

The active ingredients of extracts of the leaves of the ginkgo tree (*Ginkgo biloba*) are flavonoid glycosides, which contain, among others, (iso)quercitin glycosides, kaempferol, kaempferol-3-rhamnosides, isorhamnetin, luteoline glycosides, sitosterol glycosides and predominantly hexacyclic terpene lactones, consisting of ginkgolides A, B, C, J, M and bilobalides.

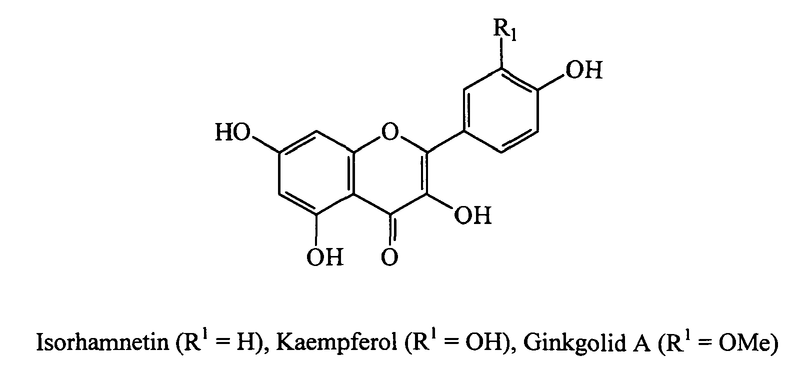

### • Camellia sinensis

Leaves of green tea contain many compounds, such as polysaccharides, volatile oils, vitamins, minerals, purines, alkaloids (e.g. caffeine) and polyphenols (catechins and flavonoids). Although all three tea types have antibacterial and free radical capturing (antioxidising) activities, the efficacy decreases substantially the darker the variety of tea is. This is due to the lower contents of anti-oxidising polyphenols remaining in the leaves. Among the various components of green tea extracts, polyphenols of the flavonoid and catechin type ("tea tannins") are the most important.

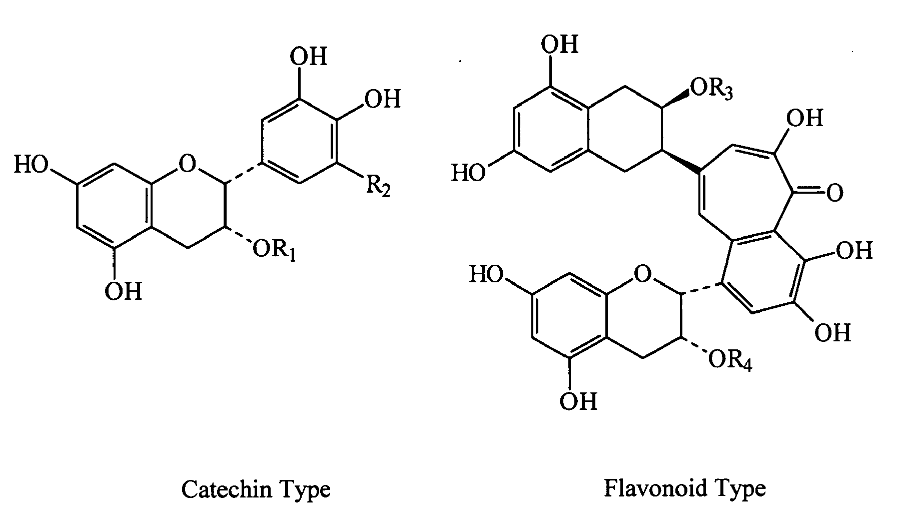

| | R1 | R2 | R3 | R4 |
|---|---|---|---|---|
| (-) Epicatechin | H | H | | |
| (-) Epigallocatechin | H | OH | | |
| (-) Epicatechin gallate | Galloyl | H | | |
| (-) Epigallocatechin gallate | Galloyl | OH | | |
| Theaflavin | | | H | H |
| Theaflavin monogallate A | | | Galloyl | H |
| Theaflavin monogallate B | | | H | Galloyl |
| Theaflavin digallate | | | Galloyl | Galloyl |

### • Olea europaea

The main constituent of the leaves of the olive tree (*Olea europaea*) is the antioxidant oleuropein, which is also the main source for hydroxytyrosol.

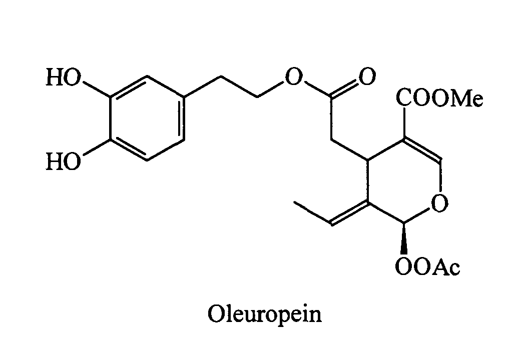

### • Glyzyrrhiza glabra

The licorice root contains glycyrrhizin, 50 times sweeter than sucrose, which encourages the production of hormones such as hydrocortisone. The extracts show an antiinflammatory action and also stimulate the adrenal cortex after steroid therapy. Main component of the extracts of *Glyzyrrhiza glabra* is glycyrrhizinic acid:

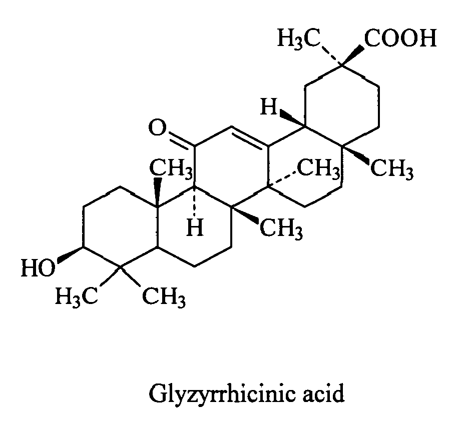

Beside the acid, also their salts, mainly the zinc salts, as well as their esters (e.g. with fatty alcohols or sterols) can be employed.

### • Vaccinium myritillus

Extracts of blueberries (*Vaccinium myrtillus*) comprise a mixture of at least 15 different anthocyanosides, like the following.

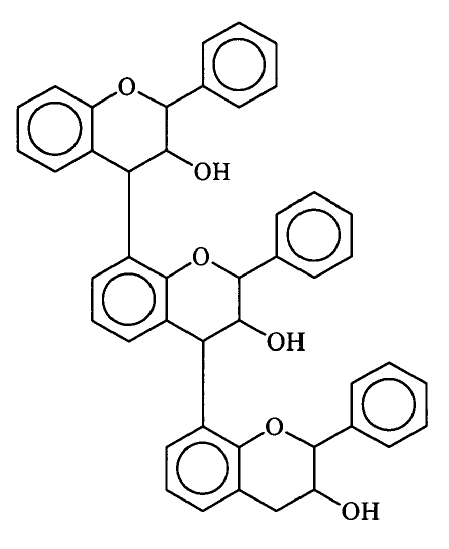

Usually, extracts of Vaccinium comprise 20 to 25 % b.w. anthocyanosides, 5 to 10 % b.w. tannins and small amounts of various alkaloids like myrtin and epimyrtin, phenolic acids and glycosides of quercitrin, isoquercitrin and hyperosid.

### • Trifolium pratense

The main active principles of red clover (*Triflolium pratense*) are isoflavones, e.g. daidzein, genestein, formononentin and biochanin as well as their glucosides, like ononin or sissostrin:

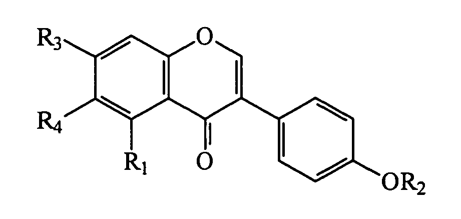

| Isoflavonglucosides | R₁ | R₂ | R₃ | R₄ |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### • Litchi sinensis

Extracts of pericarps of Litchi (*Litchi sinensis*) are well known for their high content of flavon derivatives, e.g. 2-phenyl-4H-1-benzopyrans, flavanen, flavan-3-ols (catechins, catechin oligomeren), flavan-3,4-diols (leucoanthocyaniden), flavons, flavonols and flavonons. The main component, however, represent condensed tannins, so-called procyanodols (OPC). These compounds comprise 2 to 8 monomers of the catechin or epicatechin-type, like procyanidins, proanthocynidins, procyanidoel, oligoprocyanidins, leucoanthocyanidins, leucodelphinins, leucocyanins and anthocyanogens. OPC, mainly the preferred proanthocyanidin A2 (OPC A2) behave like vitamin P, especially with respect to MMP inhibition.

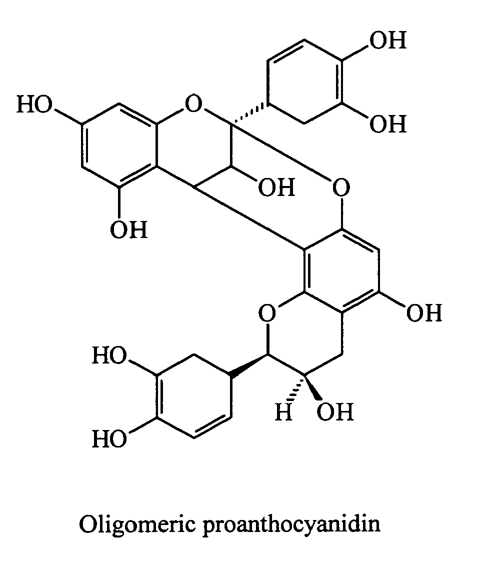

### • Vitis vinifera

The main actives of grape vine (*Vitis vinifera*) are polyphenols of the OPC type.

### • Brassica oleracea

The main active principles of cauliflower (*Brassica oleracea*) are amino acids, especially methionin and cystein, and glucosinolates like e.g. glucoraphanin.

### • Punica granatum

The main active principles of grenadine (*Punica granatum*) are sugars, citric acid and delphinidin-1,2-glykoside or its aglykon.

### • Petroselinium crispum

Main constituent of the fatty oil of parsil (*Petroselinium crispum*) is petroselinic acid. The extracts, however, show high contents of apiol (1-allyl-2,5-dimethoxy-3,4-(methylendioxy)benzol), and in addition of apiin, myristicin, pinen und selinen.

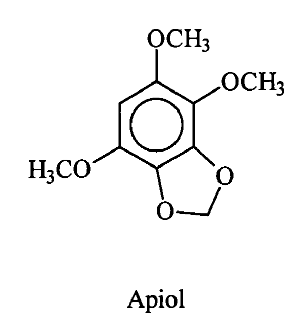

### • Centella asiatica

Main constituents of *Centella asiatica* are high condensed naphthenic acids, especially asiatica acid, madecassica acid and their glycosides.

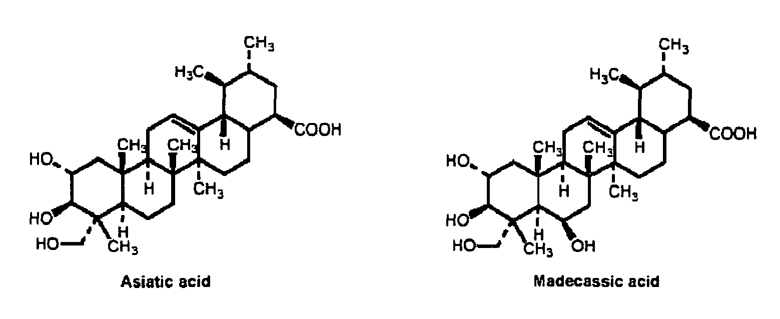

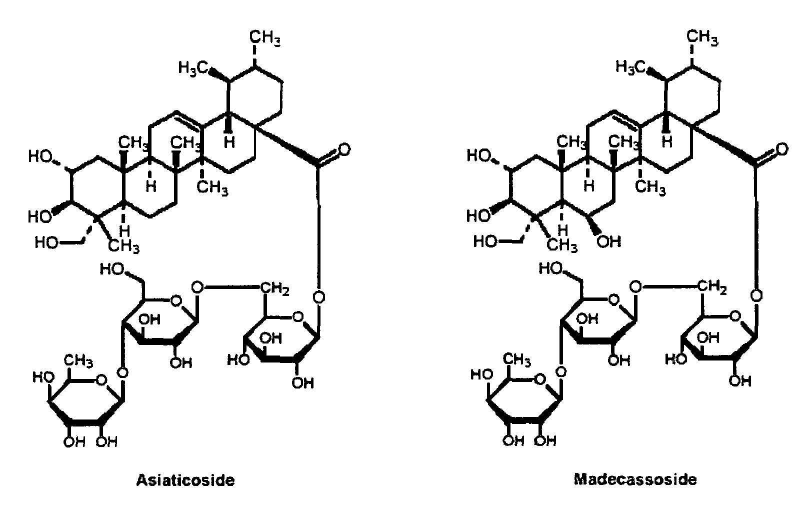

### • Passiflora incarnata

Extracts of passion flower *(Passiflora incarnata*) are rich in flavons of the apigenin and luteolin type and their C-glycosides:

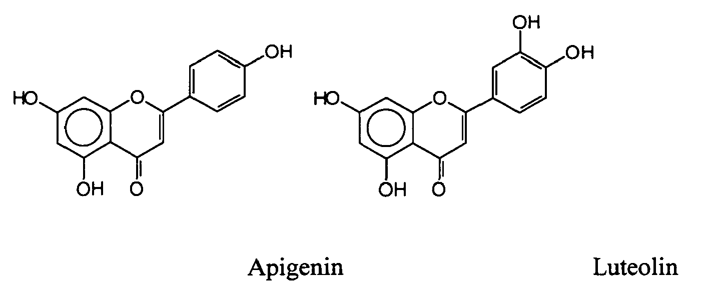

In addition they comprise 2"-B-D-glucosides, schaftosides and iso-schaftosides, isovitexin, isoorientin, vicenin-2, incenin-2, daponanin and trace elements like calcium, phosphor and iron.

### • Medicago sativa

Extracts of Alfalfa (*Medicago sativa)* are rich in isoflavons ,e.g. daidzein, genestein, formononetin, biochanin A und tricin :

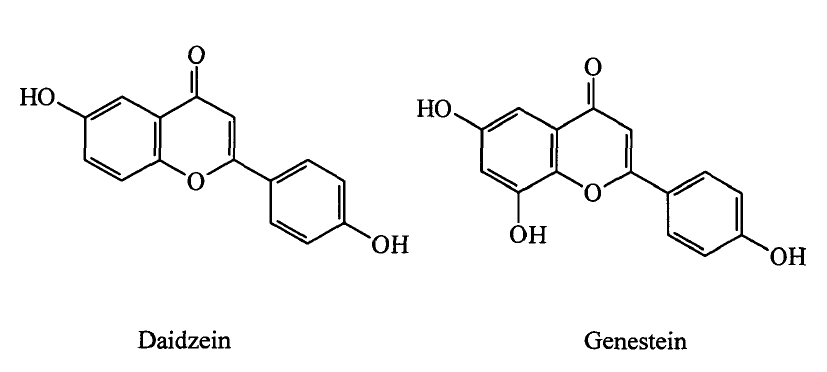

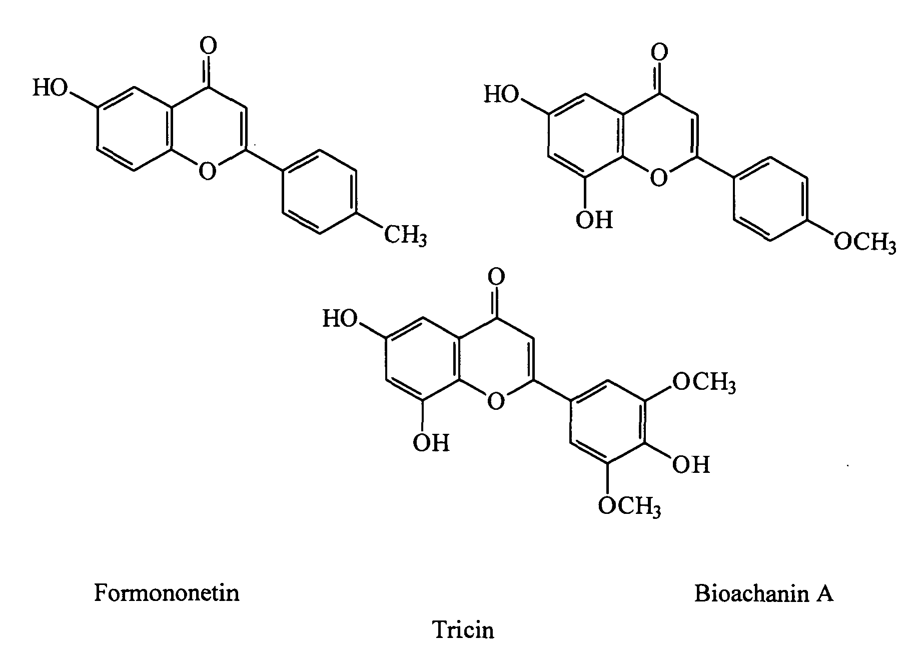

### • Valeriana officinalis

The main constituents of extracts of *Valeriana officinalis* are valeric acid, valerianone and borneol esters.

### • Aesculus hippocastanum

Main ingredients of horse chestnuts (Aesculus hippocastanum) are saponins and escin, which is a mixture of two glycosides, whose aglycons are derived from proteoescigenin, while the sugars represent either glucoronic acid of two molecules D-glucose. Said glycosides differ in the acyl groups in the C22-position.

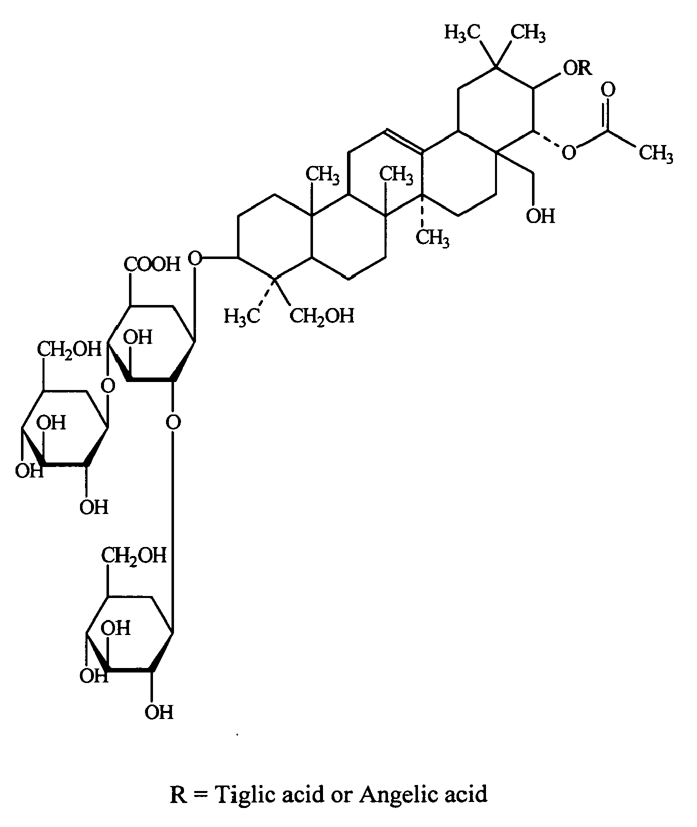

While α-escin represents an amorphous powder, which melts between 225 and 227 °C and is easily soluble in water, β-escin (which is also called flogencyl) forms flakes, which are practically water-insoluble, but can be dissolved in alcohol.

### • Salix alba

Main constituents of *Salix alba* are phenolic glycosides and especially salicylates like salicin, salicortin and tremulacin:

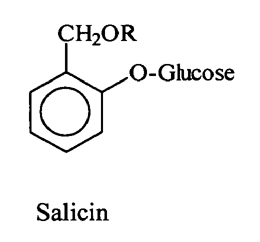

### • Harpagophytum procumbens

The main active principles of devil's claw (*Harpagophytum procumbens*) are iridoidglucosides, harpagosides, harpagides and procumbides.

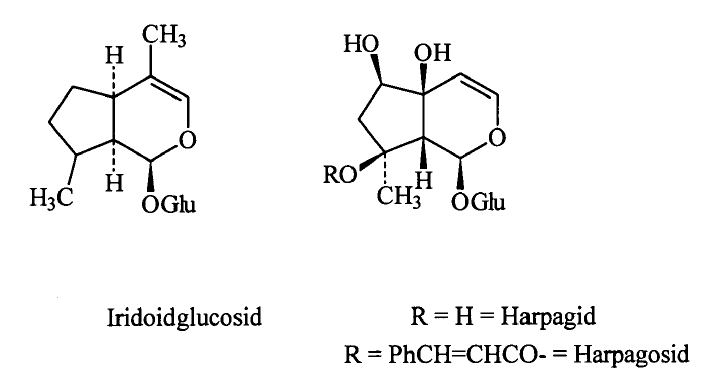

In addition, one fmds stachylose, free and glycosylated phytosterols (e.g. β-sitosterol), flavonoides (e.g. kaempferol, luteolin), phenolic acids und glycosidic phenylpropanoicacid esters (e.g. verbacosides, isoacteosides).

Instead of the botanical extracts also a purified fraction of their active principles can be used, as for example isoflavones which are derived from soy.

### Industrial application

Typical examples for personal and home care compositions in the form of liquids, emulsions, sticks, creams, lotions or even applied on wipes, which use anionic, non-ionic or amphoteric surfactants, and preferably alkyl ether sulfates (SLES) are bath and shower formulations, shampoos, soaps, facial cleansers, body scrubs, liquid dishwashing agents, liquid laundry detergents and the like. In a preferred embodiment, said compositions comprise 0.001 to 15, preferably 0.01 to 10, and more preferably, 0.5 to 2 % b.w. of the pre-mixes according to the invention. The preferred premixes comprise alkyl ether sulfates ("sodium laureth sulfate" SLES) as an anionic surfactant. Typical examples are:
- Herbalis^{®} SLES Bitter Orange - Sodium Laureth Sulfate (and) *Citrus Aurantium Amara*
- Herbalis^{®} SLES Ginkgo - Sodium Laureth Sulfate (and) *Ginkgo Biloba*
- Herbalis^{®} SLES Green Tea - Sodium Laureth Sulfate (and) *Camellia Sinensis*
- Herbalis^{®} SLES Soy - Sodium Laureth Sulfate (and) *Soy Isoflavones*

Preferably, the surfactants are present in amounts of 13 to 15 % b.w., while the amount of botanical extracts counts about 0.02 to 1 % b.w. Suitable preservatives are phenoxy ethanol, parabens, sodium benzoate and the like.

### Examples

### Examples 1 to 3, Comparative Examples C1 to C3

Samples of shampoo showing 0.5, 1 or 1.5 % b.w. of Herbalis^{®} SLES Ginkgo according to the present invention (Examples 1 to 3) were compared with a set of samples of shampoo containing ginkgo extracts dissolved in an organic medium (propylene glycol, Comparative examples C1 to C3) at 20 °C. The Gardner colour of the products and their viscosity according to Brookfield (RVT, spindle 3, 10 Upm) were determined. The results are shown in Table 1:

**Table 1**

| **Colour and Viscosity** | | | | | | |
|---|---|---|---|---|---|---|
| | **1** | **2** | **3** | **C1** | **C2** | **C3** |
| Concentration [% b.w.] | 0.5 | 1.0 | 1.5 | 0.5 | 1.0 | 1.5 |
| Gardner colour | 1.1 | 1.2 | 1.1 | 2.4 | 2.8 | 3.4 |
| Viscosity [mPas] | 2,000 | 1,900 | 1,900 | 1,600 | 1,300 | 1,030 |

As one can see, the compositions according to the present invention show a better colour and a higher and more stable viscosity. Especially the latter effect provides an additional advantage to the customer since the final formulation needs less thickener.

In the following, examples are given for personal and home care compositions comprising the pre-mixes according to the invention:

### Example 4

### Shampoo against greasy hair

| **Compound** | **Concentration [b.w.]** |
|---|---|
| Texapon^{®} N 70 **Sodium Laureth Sulfate** | 18.6 |
| Dehyton^{®} PK 45 **Cocamidopropyl Betaine** | 5.0 |
| Sodium Chloride | 1.5 |
| Herbalis^{®} SLES Green Tea **Sodium Laureth Sulfate (and) Camellia Sinensis** | 1.0 |
| Water, preservative | ad 100 |

### Example 5

### Nutritive Shower Gel

| **Compound** | **Concentration [b.w.]** |
|---|---|
| Texapon^{®} N 70 **Sodium Laureth Sulfate** | 32.0 |
| Plantapon^{®} LGC Sorb **Sodium Coco Glucoside Carboxylate** | 10.0 |
| Lamesoft^{®} TM Benz **Coco Glucosides (and) Oleyl Oleate (and) Sodium Benzoate** | 5.0 |
| Arlypon^{®} F **Laureth-2** | 0.5 |
| Sodium Chloride | 3.75 |
| Herbalis^{®} SLES Soy **Sodium Laureth Sulfate (and) Soy Isoflavones** | 1.0 |
| Water, preservative | ad 100 |

### Example 6

### Hand washing paste

| **Compound** | **Concentration [b.w.]** |
|---|---|
| Texapon^{®} N 70 **Sodium Laureth Sulfate** | 12.0 |
| Plantacare^{®} 1200 UP **Coco Glucosides** | 3.0 |
| Arlypon^{®} F **Laureth-2** | 0.5 |
| Sodium Chloride | 2.2 |
| Herbalis^{®} SLES Ginkgo **Sodium Laureth Sulfate (and) Ginkgo Biloba** | 1.5 |
| Water, preservative | ad 100 |

### Example 7

### Liquid manual dish washing composition

| **Compound** | **Concentration [b.w.]** |
|---|---|
| Maranil^{®} Paste A55 **Sodium Alkylbenzene sulfonate** | 29.8 |
| Texapon^{®} NSO **Sodium Laureth Sulfate** | 12.8 |
| Citric Acid | 0.1 |
| Herbalis^{®} SLES Bitter Orange **Sodium Laureth Sulfate (and) Citrus Aurantium Amara** | 1.0 |
| Water, preservative | ad 100 |

### Example 8

### Liquid laundry fine fabrics detergent

| **Compound** | **Concentration [b.w.]** |
|---|---|
| Texapon^{®} N 70 **Sodium Laureth Sulfate** | 6.8 |
| Demelan^{®} CS 30 **Potassium cocoate** | 2.0 |
| Dehydol^{®} LT7 90 **Laureth-712** | 3.9 |
| Glucopon^{®} 600 CS UP **Coco Glucosides** | 3.0 |
| Dehyton^{®} PK **Cocamidopropyl Betain** | 9.2 |
| Lamepon^{®} S **Potassium Cocoyl-Hydrolysed Collagen** | 2.0 |
| Euperlan^{®} PK 3000 AM **Glycol Distearate (and) Laureth-4 (and) Cocamidopropyl Betaine** | 1.0 |
| Sodium Chloride | 1.3 |
| Herbalis^{®} SLES Green Tea **Sodium Laureth Sulfate (and) Camellia Sinensis** | 1.5 |
| Water, preservative | ad 100 |

## Claims

1. Pre-mixes for personal and home care compositions, consisting of
(a) 5 to 70 % b.w. anionic, non-ionic and/or amphoteric surfactants,
(b) 0.01 to 10 % b.w. botanical extracts, and
(c) 0 to 1 % b.w. preservatives
under the condition that the amounts add with water to 100 % b.w.

2. Pre-mixes according to claim 1, **characterised in that** said anionic surfactants are selected from the group consisting of soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, alkyl oligoglucoside sulfates, protein fatty acid condensates, alkyl - (ether) phosphates and their mixtures.

3. Pre-mixes according to claim 2, **characterised in that** said anionic surfactants represent alk(en)yl ether sulfates corresponding to formula **(I),**
**R¹(OCH₂CHR²)ₙOSO₃X** **(I)**
in which R¹ is a linear or branched alkyl and/or alkenyl group containing 6 to 22 carbon atoms, R² is hydrogen or methyl, n is a number from 1 to 5 and X is alkali, alkaline earth, ammonium, alkylammonium, alkanolammonium or glucammonium.

4. Pre-mixes according to claim 1, **characterised in that** said non-ionic surfactants are selected from the group consisting of products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group; C_{12/18} fatty acid monoesters and diesters of addition products of 1 to 30 mol ethylene oxide onto glycerol; glycerol mono- and diesters and sorbitan mono- and diesters of saturated and unsaturated fatty acids containing 6 to 22 carbon atoms and ethylene oxide addition products thereof; addition products of 15 to 60 mol ethylene oxide onto castor oil and/or hydrogenated castor oil; polyol esters; addition products of 2 to 15 mol ethylene oxide onto castor oil and/or hydrogenated castor oil; C₄-C₂₂ alk(en)yl oligoglycosides; partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose); mono-, di and trialkyl phosphates and mono-, di- and/or tri-PEG-alkyl phosphates and salts thereof; wool wax alcohols; polysiloxane/polyalkyl polyether copolymers and corresponding derivatives; mixed esters of pentaerythritol, fatty acids, citric acid and fatty alcohol and/or mixed esters of C₆₋₂₂ fatty acids, methyl glucose and polyols, preferably glycerol or polyglycerol, polyalkylene glycols, glycerol carbonate and their mixtures.

5. Pre-mixes according to claim 4, **characterised in that** said non-ionic surfactants represent alk(en)yl polyalkylene glycol ethers corresponding to formula **(II),**
**R³(OCH₂CHR⁴)ₘOH** **(II)**
in which R³ is a linear or branched alkyl and/or alkenyl group containing 6 to 22 carbon atoms, R⁴ is hydrogen or methyl, and m is a number from 1 to 20.

6. Pre-mixes according to claim 4, **characterised in that** said non-ionic surfactants represent alk(en)yl oligoglycosides corresponding to formula **(III),**
**R⁵O[G]ₚ** **(III)**
where R⁵ is an alkyl or alkenyl radical having from 4 to 22 carbon atoms, G is a sugar unit having 5 or 6 carbon atoms and p is a number from 1 to 10.

7. Pre-mixes according to claim 1, **characterised in that** said amphoteric surfactants are selected from the group consisting of alkyl betaines, alkylamido betaines, imidazolins, amphoglycinates and their mixtures.

8. Pre-mixes according to any of claims 1 to 7, **characterised in that** said botanical extracts are selected from the plants which may be of interest for the Personal and Home Care industry selected from the group consisting of *Glycine max, Ginkgo biloba, Camellia sinensis, Olea europaea, Glyzyrrhiza glabra, Vaccinium myrtillus, Trifolium pratense, Litchi sinensis, Vitis vinifera, Brassica oleracea, Punica granatum, Petroselinium crispum, Centella asiatica, Passiflora incarnata, Medicago sativa, Valeriana officinalis, Aesculus hippocastanum, Citrus aurantium amara, Salix alba Hapagophytum procumbens, Calendula officinalis, Chamomila recutita, Panax ginsing, Rosa canina, White musc* and their mixtures.

9. Use of pre-mixes according to claim 1 for making personal or home care compositions.

10. Use according to claim 9, **characterised in that** said pre-mixes are added to said personal or home care compositions in amounts of 0.001 to 15 % b.w.
